# NEUE EUROPÄISCHE PATENTSCHRIFT

(11) **EP 2 094 210 B2**
(45) Veröffentlichungstag und Bekanntmachung des Hinweises auf die Entscheidung über den Einspruch: **08.09.2021**
(45) Hinweis auf die Patenterteilung: 20.06.2018
(21) Anmeldenummer: 07856658.5
(22) Anmeldetag: 12.12.2007
(51) Int. Cl.: A61L 15/44, A61F 13/00, A61M 35/00

(54) **MEDIZINISCHES FLÄCHENGEBILDE**
MEDICAL FABRIC
STRUCTURE PLANE MÉDICALE

(30) Priorität: 27.12.2006 DE 102006061539
(43) Veröffentlichungstag der Anmeldung: 02.09.2009
(73) Patentinhaber: Paul Hartmann AG, 89522 Heidenheim (DE)
(72) Erfinder: MALOWANIEC, Krzysztof, Daniel, 89522 Heidenheim (DE)
(74) Vertreter: Ter Meer Steinmeister & Partner
(86) Internationale Anmeldenummer: PCT/EP2007/010912
(87) Internationale Veröffentlichungsnummer: WO 2008/080532

(56) Entgegenhaltungen:
- WO-A-2005/004935
- WO-A1-2005/004935
- WO-A1-2007/068490
- DE-A1- 19 925 519
- DE-A1- 19 925 519
- DE-A1-102006 050 806
- DE-A1-102006 050 806
- DE-B3- 10 316 156
- DE-B3- 10 316 156
- FR-A1- 2 786 396
- KR-A- 20040 040 044
- KR-A- 20060 016 282
- US-A- 4 191 743
- US-A- 4 191 743
- US-A1- 2004 241 214
- US-A1- 2006 264 796
- US-A1- 2008 249 453
- J. DUTKIEWICZ: "Some Advances in Nonwoven structures for Absorbency, Comfort and Aesthetics", AUTEX Research Journal, vol. 2, no. 3, 2002, pages 153-165,
- LEE, JONG-EUN et al.: "An Infection-Preventing Bilayered Collagen Membrane Containing Antibiotic-Loaded Hyalouronan Microparticles: Physical and Biological Properties", Artificial Organs, vol. 26, no. 7 2002, pages 1-24, DOI: 10.1046/j.1525-1594.2002.06847.x Retrieved from the Internet: URL:https://onlinelibrary-wiley.com.ezprox y.lib.vt.edu/doii/full/10.1046/j.1525-1594 .2002.06847.x
- CHANG, HELEN et al.: "Moist wound healing", Dermatology Nursing, vol. 8, no. 3, 1996, page 174,
- OVINGTON et al.: "Wound Care Products: How to Choose", Advances in Skin & Wound Care, vol. 14, no. 5, 2001, pages 259-266,
- NINGTAO, MAO: "Effect of Fabric Structure on the Liquid Transport Characteristics of Nonwoven Wound Dressings", Dissertation, University of Leeds, School of Textile Industries, 2000, pages FP-275,
- JIANG S Q; ET AL: "Chemical Silver Plating on Cotton and Polyester Fabrics and its Application on Fabric Design", Textile Research Journal, vol. 76, no. 1, 1 January 2006 (2006-01-01), pages 57-65,
- Thomas: "Wound Management and Dressings", The Pharmaceutical Press page 40,111,

## Beschreibung

Die vorliegende Erfindung betrifft eine Wundauflage umfassend ein medizinisches Flächengebilde in Form einer Wundkontaktschicht, die eine erste Schicht mit einem die Wundheilung fördernden Mittel umfasst. Weiterhin betrifft die Erfindung eine Wundauflage zur Behandlung von mittel bis stark exsudierenden Wunden, sowie deren Verwendung in der modernen Wundbehandlung.

Die Heilung von Hautwunden beruht auf der Fähigkeit der Haut zur Regeneration von Epithelen sowie von Binde- und Stützgewebe. Sie ist als ein komplexes Geschehen ineinander übergreifender Zellaktivitäten gekennzeichnet, die den Heilungsprozess schrittweise vorantreiben. So werden in der Literatur unabhängig von der Art der Wunde drei wesentliche Heilungsphasen einer Wunde beschrieben. Hierzu gehört die inflammatorische oder exsudative Phase zur Blutstillung und Wundreinigung (Phase 1, Reinigungsphase), die proliferative Phase zum Aufbau von Granulationsgewebe (Phase 2, Granulationsphase) und die Differentierungsphase zur Epithelisierung und Narbenbildung (Phase 3, Epithelisierungsphase).

Zur Unterstützung der einzelnen Wundheilungsphasen sind zahlreiche Vorschläge in der Literatur beschrieben. So beschreibt beispielsweise die EP 297 828 eine Wundauflage, die eine bioaktive Substanz und ein Trägermaterial umfasst. Die bioaktive Substanz liegt in Form von wachsartigen Partikeln mit einem niedrigen Schmelzpunkt vor, die bei Kontakt mit der Haut schmelzen und die Substanz freisetzen.

Weiterhin ist mit der DE 26 50 306 ein antimikrobiell wirkender Wundverband bekannt, der ein Antibiotikum umfasst. Das Antibiotikum liegt zur Abgabe über eine lange Zeit in Form eines Kunstoffgranulates innerhalb eines offenporigen Schaummaterials vor.

Mit der WO 2005/ 004 936 ist eine Wundauflage bekannt, die wirkstoffhaltige Partikel innerhalb einer hydropilen Polyurethan-Matrix umfasst. Diese Matrix umfasst keine Fasern oder Fasermaterialien.

Mit der DE 10 2005 004 924 wird eine Vorrichtung zur Behandlung von trophischen Störungen in einer Haut oder in einer Wunde mittels Kohlenstoffdioxid beschrieben. Die Vorrichtung umfasst u.a. Mittel zur Erzeugung von Kohlendioxid, eine wässrige Phase zur Lösung des Kohlenstoffdioxids und ein Depot für die wässrige Phase. Mit diesem Schutzrecht ist auch eine Vorrichtung bekannt, die innerhalb eines aus einem superabsorbierenden Polymer bestehenden Depots partikelförmige Salze umfasst, aus denen das Kohlendioxid freigesetzt wird. Diese Partikel sind nicht mit einem Fasermaterial verbunden.

Darüber hinaus sind zahlreiche wirkstoffhaltige Pflastersysteme als Transdermal Therapeutische Systeme (TTS) bekannt, die Wirkstoffe sind Bestandteil einer meist klebenden Matrix oder Films, die/ der über einen längeren Zeitraum die Wirkstoffe über die Haut in den Körper eines Anwenders einbringt. Diese Darreichungsform stellt eine Alternative zur oralen Einnahme eines Wirkstoffs dar.

Aufgabe der vorliegenden Erfindung ist es, ein alternatives und verbessertes Mittel zur Abgabe von Wirkstoffen an eine Wunde oder die die Wunde umgebende Haut bereitzustellen. Weiterhin soll eine Wundauflage zur Verfügung gestellt werden, die in der Lage ist, die menschliche Haut zu pflegen und/ oder den Heilungsprozess einer Wunde optimal zu unterstützen, indem ein Wirkstoff zur Anwendung gebracht wird. Darüber hinaus soll eine Wundauflage zur Verfügung gestellt werden, die einen hautpflegenden und/oder wundheilungsfördernden Wirkstoff und ein feuchtes Wundheilungsklima bereitstellt und die sich besonders gut an eine zu behandelnde Wunde anpasst

Gelöst werden diese Aufgaben von einer Wundauflage umfassend ein medizinisches Flächengebilde, worin das medizinische Flächengebilde eine Wundkontaktschicht ist und die Wundkontaktschicht mindestens eine erste Schicht aus einem Fasermaterial mit einer ersten im Gebrauch dem Körper zugewandten Oberseite und einer zweiten im Gebrauch dem Körper abgewandten Oberseite, und mindestens eine wundheilungsfördernde Substanz umfasst, die in Form von Partikeln vorliegt, umfasst, wobei die Partikel mit dem Fasermaterial verbunden sind und die wundheilungsfördernde Substanz in Gegenwart von wässriger Flüssigkeit, insbesondere Wundexsudat, freigesetzt wird, dadurch gekennzeichnet dass auf der zweiten Oberseite eine absorbierende Schicht angeordnet ist und die Wundauflage als diese absorbierende Schicht einen hydrophilen Polymer-Schaum umfasst.

Die Abgabe mindestens einer wundheilungsfördernden Substanz wird hierbei unter anderem durch die in der bestimmungsgemäßen Anwendung des Flächengebildes in unmittelbarer Umgebung zu den Partikeln vorhandene wässrigen Flüssigkeit oder Wundexsudat gesteuert. Je mehr Flüssigkeit oder Wundexsudat in der direkten Umgebung der Partikel vorhanden ist, desto mehr an wundheilungsfördernder Substanz wird abgegeben. Die Partikel stellen somit ein Depot für eine wundheilungsfördernde Substanz dar, das diese Substanz kontrolliert freisetzt. Damit steht beispielsweise einer zu behandelnden Wunde durch das Flächengebilde eine wundheilungsfördernde Substanz bedarfsgerecht zur Verfügung. Es kann jedoch auch vorgesehen sein, dass das Flächengebilde vor der Anwendung mittels einer wässrigen Flüssigkeit aktiviert wird und somit ein Flächengebilde bereitgestellt wird, das eine wundheilungsfördernde Substanz sofort freisetzt. Im ersten Fall ist eher eine verzögerte Abgabe von Substanz über einen längeren Zeitraum gewährleistet, wogegen im zweiten Fall eine Einmaldosis einer Substanz zur Verfügung gestellt wird. Dadurch, dass die Partikel mit dem Fasermaterial verbunden sind, wird gewährleistet, dass bei der bestimmungsgemäßen Verwendung des Flächengebildes keine Partikel in die Wunde gelangen. Nicht verwertete wundheilungsfördernde Substanz kann zudem durch einfaches Entfernen des Flächengebildes von der zu behandelnden Stelle, beispielsweise einer Wunde, vollständig entfernt werden, ohne dass überschüssige Substanz verbleibt. Somit ist ein Flächengebilde zur Verfügung gestellt, das eine erhöhte Therapiesicherheit gewährleistet. In jedem Fall sind im Zusammenhang mit der vorliegenden Erfindung als wässrige Flüssigkeit Wasser, Salzlösungen, insbesondere isotonische Kochsalzlösung oder Ringer-Lösung, oder Wundexsudat zu verstehen.

Gemäß der vorliegenden Erfindung sollen dabei die Partikel mit dem Fasermaterial verbunden sein. Hiermit soll verstanden sein, dass die Partikel mit ihrer äußeren Oberfläche mit dem Fasermaterial, insbesondere den Fasern selbst, in direktem Kontakt stehen und verbunden sind. Die Verbindung kann hierbei fest oder lösbar ausgeführt sein. Insbesondere umfasst das Fasermaterial erste Fasern, mit denen die Partikel verbunden sind. Hierbei setzen die mit den Fasern verbundenen Partikel die wundheilungsfördernde Substanz in Gegenwart von wässrigen Flüssigkeiten, insbesondere Wasser, isotonischer Kochsalzlösung, Ringerlösung oder Wundexsudat frei. Unter isotonischer Kochsalzlösung ist dabei eine 0,9 Gew.-%-ige Natriumchloridlösung zu verstehen. Eine Ringerlösung enthält gemäß der vorliegenden Erfindung ein Gemisch aus verschiedenen Salzen in Wasser, wobei in 1000 ml Ringerlösung 8,60 g Natriumchlorid, 0,30 g Kaliumchlorid, 0,33 g Kalziumchlorid-Dihydat in gereinigtem und destilliertem Wasser gelöst vorliegen.

Gemäß einer zweiten Ausführung der vorliegenden Erfindung können die Partikel in oder auf die Fasern ein- oder aufgebracht sein. Insbesondere können die Partikel auch in oder auf die das Fasermaterial umfassende Schicht ein- oder aufgebracht sein, wobei weiterhin bevorzugt die Partikel innerhalb der Schicht angeordnet sind. Insbesondere kann dabei auch vorgesehen sein, dass das Fasermaterial erste Fasern und zweite Fasern umfasst, wobei die ersten Fasern eine wundheilungsfördernde Substanz in Form von Partikeln umfassen, die in oder an die Fasern ein- oder aufgebracht ist, und weiterhin bevorzugt die zweiten Fasern frei von wundheilungsfördernden Substanzen sind. Dabei können die zweiten Fasern aus einem gleichen oder verschiedenen Material wie die ersten Fasern hergestellt sein.

Insbesondere umfasst das Fasermaterial erste und zweite Fasern, wobei die Partikel mit den ersten Fasern verbunden sind und die wundheilungsfördernde Substanz in Gegenwart von wässriger Flüssigkeit oder Wundexsudat freisetzen.

Gemäß einer weiteren Ausführung der Erfindung kann auch vorgesehen sein, dass die Partikeln in der ersten Schicht gleichmäßig verteilt sind. Dies kann sichergestellt werden, indem das Flächengebilde in einem so genannten Airlaid-Verfahren hergestellt wird, wobei die Partikel und die Fasern zusammen oder getrennt nacheinander gleichmäßig zu einer Schicht zusammengebracht werden. Es kann jedoch auch vorgesehen sein, die Partikel in Beschichtungsverfahren gleichmäßig auf das Fasermaterial oder die Schicht aufzubringen.

Des Weiteren ist gemäß der vorliegenden Erfindung vorgesehen, dass das Flächengebilde sterilisierbar ist oder das Flächengebilde ein steriles Flächengebilde ist. Zur Sterilisation können hierbei insbesondere die Dampfsterilisation oder die Strahlensterilisation mittels β-Strahlung herangezogen werden. Es ist jedoch auch möglich eine Sterilisation mittels Ethylenoxid durchzuführen.

Gemäß der vorliegenden Erfindung soll ein Flächengebilde mindestens eine wundheilungsfördernde Substanz umfassen, die in Form von Partikeln vorliegt. Als wundheilungsfördernde Substanz in Form von Partikeln umfasst ein Flächengebilde insbesondere eine feste oder zumindest halbfeste wundheilungsfördernde Substanz in Form von Partikeln oder Pulvern. In dieser Form kann die Substanz in Reinform oder als Gemisch mit einem Trägermaterial vorliegen. Es kann jedoch auch vorgesehen sein, dass die Substanz in Form von Kern-Hülle-Partikeln oder Kern-Hülle-Pulvern vorliegen. Hierbei ist die freizusetzende Substanz von einer Hülle umgeben, die in Gegenwart von wässrigen Flüssigkeiten für die Substanz durchlässig wird oder sich auflöst. Somit steht die Hülle als Trägermaterial zur Verfügung. Falls jedoch die zu verabreichende Substanz als Flüssigkeit oder als Lösung, Gel oder sonstige eher flüssige Form vorliegt, umfassen die Partikel in jedem Fall die flüssige Substanz und ein Trägermaterial für die flüssige Substanz. Dieses Trägermaterial wiederum kann als Hülle vorliegen oder die wundheilungsfördernde Substanz in eine feste Form überführen. Damit umfasst gemäß einem weiterführenden Gedanken auch ein Flächengebilde ein Fasermaterial und eine wundheilungsfördernde Substanz, wobei die Substanz in Form von Partikeln vorliegt, die die wundheilungsfördernde Substanz und ein Trägermaterial für die Substanz umfassen. Hierbei kann das Trägermaterial in Bezug auf das Fasermaterial aus einem gleichen oder verschiedenen Material gefertigt sein, das heißt, dass das Fasermaterial Fasern umfasst, die aus dem selben Material gefertigt sind wie das Trägematerial für die freizusetzende Substanz. Insbesondere ist gemäß der vorliegenden Erfindung vorgesehen, dass das Fasermaterial Fasern umfasst, die aus demselben Material bestehen, wie das Trägermaterial der Partikel oder die Partikelhülle.

Hierbei können insbesondere Partikel verwendet werden, die als Mikrokapsel oder Mikropartikel vorliegen. Gemäß der vorliegenden Erfindung werden Partikel als Mikrokapseln oder Mikropartikel betrachtet, wenn diese Partikel oder Kapseln einen mittleren Teilchendurchmesser von mindestens 1 µm und höchstens 1000 µm aufweisen. Damit ist auch ein Flächengebilde umfassend mindestens eine wundheilungsfördernde Substanz in Form von Partikel Gegenstand der vorliegenden Erfindung, die einen mittleren Partikeldurchmesser von 1 bis 1000 µm, insbesondere von 1 bis 700 µm, insbesondere von 10 bis 500 µm und ganz besonders bevorzugt von 50 bis 500 µm aufweisen.

Insbesondere sind solche Mikrokapseln oder -partikel geeignet, die ein Trägermaterial für die wundheilungsfördernde Substanz und die wundheilungsfördernde Substanz umfassen. Weiterhin bevorzugt sind solche Flächengebilde, die Partikel aufweisen, die aus einer Hülle und einem Kern bestehen und die Partikel mittels der Hülle mit dem Fasermaterial oder den Fasern verbunden sind. Dabei ist die Hülle derart ausgeführt, dass sie in Gegenwart von wässriger Flüssigkeit oder Wundexsudat für die Substanz durchlässig wird, so dass die Substanz aus dem Kern freigesetzt wird.

Dadurch dass die wundheilungsfördernde Substanz durch die Mikrokapselhülle eingeschlossen sind, wird erreicht, dass bei der Lagerung eines Flächenmaterials keine Wechselwirkung zwischen dem Fasermaterial oder anderer in der näheren Umgebung vorhandenen Materialien stattfinden kann. Die Hülle stellt gegenüber der wundheilungsfördrnden Substanz eine inerte Umgebung dar, die keine Wechselwirkung mit der Substanz eingeht.

In einer weiteren Ausführungsform weist das Flächengebilde Partikel auf, die ein Trägermaterial umfassen, das aus einem gelbildenden Polymer besteht. Als gelbildendes Polymer sind im Zusammenhang mit der vorliegenden Erfindung insbesondere Polymere ausgewählt aus der Gruppe der Polyacrylate, Alginate, Chitin oder dessen Derivate, Cellulose oder deren Derivate oder Kombinationen hiervon geeignet. Diese gelbildenden Polymere sind als Trägermaterialien besonders geeignet, da sie eine gute Bildung mit den Fasern oder dem Fasermaterial eingehen.

Gemäß einer weiteren Ausführung der vorliegenden Erfindung umfasst ein Flächengebilde eine erste und eine von der ersten verschiedene zweite wundheilungsfördernde Substanz. Dabei kann vorgesehen sein, dass die erste und die zweite Substanz als Gemisch in Form von Partikeln eingesetzt werden. Hierbei kann jede erste Substanz in Reinform oder zusammen mit einem ersten Trägermaterial in Form erster Partikel und die zweite wundheilungsfördernde Substanz in Reinform oder zusammen mit einem Trägematerial in Form zweiter Partikel vorliegen. Es kann jedoch auch vorgesehen sein, das die erste und die zweite wundheilungsfördernde Substanz als Gemisch oder als Gemisch zusammen mit einem Trägermaterial in Form erster Partikel vorliegen. Weiterhin kann vorgesehen sein, dass die erste wundheilungsfördernde Substanz in Form von Partikeln vorliegt und die zweite wundheilungsfördernde Substanz in oder auf die Fasern des Fasermaterials ein oder aufgebracht ist oder homogen in oder auf den Fasern vorliegt.

Weiterhin bevorzugt kann vorgesehen sein, dass das Flächengebilde eine erste und eine zweite wundheilungsfördernde Substanz umfasst, wobei die Freisetzung der ersten wundheilungsfördernden Substanz in Gegenwart von wässriger Flüssigkeit insbesondere Wundexsudat, zu einem ersten Zeitpunkt und/ oder über einen ersten Zeitraum erfolgt und die Freisetzung der zweite wundheilungsfördernde Substanz in Gegenwart von wässriger Flüssigkeit, insbesondere Wundexsudat, zu einem zweiten, von dem ersten verschiedenen Zeitpunkt und/ oder über einen zweiten, von dem ersten verschiedenen Zeitraum erfolgt. Hierbei kann vorgesehen sein, das die erste und die zweite Substanz in Form von Partikeln vorliegt, wobei die erste Substanz in Form von Partikeln vorliegt, die eine Hülle und einen Kern aufweisen, wogegen die zweite Substanz in Reinform als Partikel oder Pulver vorliegt. In jedem Fall sind zumindest die ersten Partikel mit der ersten Substanz mit dem Fasermaterial oder den Fasern verbunden.

Gemäß der vorliegenden Erfindung umfasst das Flächengebilde mindestens eine erste Schicht aus einem Fasermaterial, mit dem mindestens eine wundheilungsfördernde Substanz in Form von Partikeln verbunden ist. Insbesondere handelt es sich bei diesem Flächengebilde, um ein nicht klebendes und/oder nicht wundverklebendes Flächengebilde, insbesondere um eine nicht klebende und/ oder nicht wundverklebende Wundkontaktschicht. Insbesondere umfasst das Fasermaterial dabei erste Fasern, die mit den Partikeln verbunden sind, wobei die Partikel die wundheilungsfördernde Substanz in Gegenwart von wässriger Flüssigkeit oder Wundexsudat freisetzen. Weiterhin bevorzugt umfasst das Fasermaterial erste und zweite Fasern oder ein Gemisch verschiedener Fasern.

Gemäß einer bevorzugten Ausführungsform, kann vorgesehen sein, dass die erste Schicht selbst mehrschichtig ist. In diesem Fall kann vorgesehen sein, dass lediglich eine der Schichten Fasern aufweist, die mit wundheilungsfördernden Substanz in Form von Partikeln verbunden sind. Alternativ können auch wundheilungsfördernde Substanzen in Form von Partikeln in gleicher oder verschiedener Art in mehreren Schichten untergebracht sein.

Weiterhin bevorzugt umfasst ein Flächengebilde auch eine erste und mindestens eine zweite Schicht. Diese zweite Schicht ist auf der im anwendungsgerechten Zustand einer Wunde zugewandten Oberseite der ersten Schicht angeordnet. Insbesondere handelt es sich bei dieser zweiten Schicht ebenfalls um eine Schicht aus einem Fasermaterial. Hierbei kann vorgesehen sein, dass die erste Schicht erste Fasern und die zweite Schicht zweite Fasern umfasst.

In einer weiteren Ausführung der Erfindung ist vorgesehen, dass die erste Schicht, die ein Fasermaterial und eine wundheilungsfördernde Substanz in Form von Partikeln umfasst, ein Nonwoven, ein Gestrick, ein Gewirk oder ein Gewebe ist.

Insbesondere kann als Nonwoven ein Nonwoven aus Stapelfasern eingesetzt werden. Insbesondere sind weiterhin solche Nonwoven bevorzugt einsetzbar, die mittels Wasserstrahlen und/ oder thermischer Verfestigung stabilisiert oder verfestigt sind. Hierbei sind weiterhin bevorzugt solche Stapelfasern einzusetzen, die eine Faserlänge von 3 bis 60 mm aufweisen. Diese Stapelfasern können insbesondere aus organischen Fasern bestehen, die synthetische und/ oder natürliche Polymere umfassen. Ganz besonders bevorzugt ist ein Fasermaterial, das aus Viskosefasern, Cellulosefasern, Alginatfasern, Acetatfasern, Polyersterfasern, Polyamidfasern, Polyethylenfasern, Polypropylenfasern oder Kombinationen hiervon besteht. Weiterhin bevorzugt hiervon ist ein Fasermaterial, das aus Viskosefasern und/ oder Cellulosefasern besteht.

Für die Herstellung der medizinischen Flächengebilde oder der Wundkontaktschicht haben sich Stapelfasern einer Länge von 3 bis 60 mm bewährt. Als Stapelfasern werden Fasern einer definierten Länge verstanden, welche Chemiefasern, also aus natürlichen oder synthetischen Polymeren industriell hergestellte Fasern, und natürliche Fasern sein können. Wenn Chemiefasern als Stapelfasern verwendet werden, so können diese in vorteilhafter Weiterbildung auch Längen von 15 bis 40 mm, insbesondere von 15 bis 25 mm aufweisen. Die Länge von natürlichen Stapelfasern, wie beispielsweise Baumwollfasern betragen vorteilhafterweise 9 - 15 mm, insbesondere etwa 12 mm.

Die vorstehend erwähnten Chemiefasern als Stapelfasern können in vorteilhafter Weise Mikrostapelfasern umfassen oder Mikrostapelfasern sein. Der Begriff Mikrostapelfasern bedeutet hierbei, dass diese Fasern eine Stärke von < 1 dtex haben.

Mikrostapelfasern können in vorteilhafter Weise Polyester(PES)-Fasern oder Viskosefasern sein (Viskosefasern bestehen zwar aus natürlichen Zellulosemolekülen, die aber synthetisch zur Bildung von Fasern verarbeitet werden).

Wenn Mikrostapelfasern verwendet werden, so sind sie an ihrer Oberfläche vorteilhafter Weise hydrophiliert, um ein gutes Flüssigkeitsaufnahmevermögen bereit zu stellen. Der Anteil der Mikrostapelfasern beträgt in vorteilhafter Weise 15 bis 85 Gew.-%, insbesondere 15 bis 65 Gew.-% und weiter insbesondere 20 bis 30 Gew.-% innerhalb der ersten Schicht des Flächengebildes oder der Wundkontaktschicht. Die Angabe der Gewichtsanteile der Fasern ist stets bezogen auf das trockene Flächengebilde oder die trockene Wundkontaktschicht jeweils mit den verbundenen Partikeln zu verstehen.

Wie bereits erwähnt, kann es sich als vorteilhaft erweisen, wenn in der ersten Schicht bis zu 75 Gew.-%, insbesondere 15 bis 65 Gew.-% und weiter insbesondere 50 bis 65 Gew.-% Baumwollfasern enthalten sind. Hierbei handelt es sich vorzugsweise um Baumwollkämmlinge. In Weiterbildung der Erfindung wird vorgeschlagen, dass das Flächengebilde oder die Wundkontaktschicht zusätzlich wärmeschmelzbare Bindefasern, ebenfalls als Stapelfasern umfasst, insbesondere in einem gewichtsprozentualen Anteil von 10 bis 20 Gew.-%, insbesondere von 10 bis 18 Gew.-% und weiter insbesondere von 10 bis 15 Gew.-%.

Bei diesen Bindefasern kann es sich in vorteilhafter Weise um Mehrkomponentenfasern, insbesondere Bikomponentenfasern handeln, mit insbesondere einer Mantel/Kern-Anordnung der beiden Komponenten. Diese Mehrkomponentenfasern haben in vorteilhafter Weise eine Faserstärke von 1,3 - 10 dtex, insbesondere von 1,3 bis 3,0 dtex, und eine Faserlänge von 3 bis 60 mm, insbesondere von 40 bis 60 mm. Bei bevorzugt zu verwendenden Bikomponentenfasern kann es sich um Polyester (PES) / Copolyester (CO-PES)-Bikomponentenfasern handeln.

Es erweist sich in jedem Fall als vorteilhaft, wenn der Schmelzpunkt der wärmeschmelzbaren Bindefasern oder der niedrig schmelzenden Komponente der Mehrkomponentenfasern als Bindefasern geringer ist als der Schmelzpunkt von zusätzlich verwendeten Stapelfasern oder Mikrostapelfasern.

Ein bevorzugtes Nonwoven umfasst eine Zusammensetzung von verschiedenen Fasern. Diese Zusammensetzung umfasst zu 20 bis 30 Gew.-% Mikrostapelfasern, zu 10 bis 20 Gew.-% Bindefasern und zu 50 bis 70 Gew.-% Baumwollfasern.

Die Flächengebilde oder die Wundkontaktschichten können zur Verfestigung wasserstrahlvernadelt sein, was insbesondere nur oberflächennahe Bereiche der Fasern erfassen oder auch eine durchgehende Vernadelung der Flächengebilde oder der Wundkontaktschichten bedeuten kann. Die Flächengebilde oder die Wundkontaktschichten können aber auch thermoverfestigt sein. Die Verfestigungsschritte der Wasserstrahlvernadelung oder Thermoverfestigung können einzeln oder vorzugsweise kombiniert vorliegen.

Als wundheilungsfördernde Substanz in Form von Partikeln umfasst ein Flächengebilde, insbesondere eine Wundkontaktschicht oder eine Wundauflage insbesondere eine feste oder zumindest halbfeste Substanz. Falls jedoch die wundheilungsfördernde Substanz eher in einer flüssigen Form vorliegt, umfassen die Partikel in jedem Fall ein Trägermaterial und eine flüssige Substanz.

Im Zusammenhang mit der vorliegenden Erfindung ist insbesondere mindestens eine Substanz ausgewählt aus der Gruppe der Vitamine oder Provitamine, Carotinoide, Analgetika, Antiseptika, Hämostyptika, Antihistaminika, antimikrobiellen Metalle oder deren Salze, pflanzlichen wundheilungsfördernden Substanzen oder Substanzgemische, Pflanzenextrakte, Enzyme, Wachstumsfaktoren, Enzyminhibitoren sowie Kombinationen hiervon, zu verwenden.

Als Analgetika sind insbesondere nicht-steroidale Analgetika insbesondere Salicylsäure, Acetylsalicylsäure und deren Derivate z.B. Aspirin®, Anilin und dessen Derivate, Acetaminophen z.B. Paracetamol®, Antranilsäure und deren Derivate z.B. Mefenaminsäure, Pyrazol oder dessen Derivate z.B. Methamizol, Novalgin®, Phenazon, Antipyrin®, Isopropylphenazon und ganz besonders bevorzugt Arylessigsäuren sowie deren Derivate, Heteroarylessigsäuren sowie deren Derivate, Arylpropionsäuren sowie deren Derivate und Herteroarylpropionsäuren sowie deren Derivate z.B. Indometacin®, Diclophenac®, Ibuprofen®, Naxoprophen®, Indomethacin®, Ketoprofen®, Piroxicam® geeignet.

Als Wachstumsfaktoren sind insbesondere zu nennen: aFGF (Acidic Fibroplast Growth Factor), EGF (Epidermal) Growth Factor), PDGF (Platelet Derived Growth Factor), rhPDGF-BB (Becaplermin), PDECGF (Platelet Derived Endothelial Cell Growth Factor), bFGF (Basic Fibroplast Growth Factor),TGF α (Transforming Growth Factor alpha), TGF β (Transforming Growth Factor beta), KGF (Keratinocyte Growth Factor), IGF1/IGF2 (Insulin-Like Growth Factor) und TNF (Tumor Necrosis Factor).

Als Vitamine oder Provitamine sind insbesondere die fettlöslichen oder wasserlöslichen Vitamine Vitamin A, Gruppe der Retinoide, Provitamin A, Gruppe der Carotenoide, insbesondere β-Carotin, Vitamin E, Gruppe der Tocopherole, insbesondere α-Tocopherol, β-Tocopherol, γ-Tocopherol, δ-Tocopherol und α-Tocotrienol, β-Tocotrienol, γ-Tocotrienol und δ-Tocotrienol, Vitamin K, Phyllochinon insbesondere Phytomenadion oder pflanzliches Vitamin K, Vitamin C, L-Ascorbinsäure, Vitamin B1, Thiamin, Vitamin B2, Riboflavin, Vitamin G, Vitamin B3, Niacin, Nikotinsäure und Nikotinsäureamid, Vitamin B5, Pantothensäure, Provitamin B5, Panthenol oder Dexpanthenol, Vitamin B6, Vitamin B7, Vitamin H, Biotin, Vitamin B9, Folsäure sowie Kombinationen hiervon geeignet.

Als Antiseptikum ist ein solches Mittel zu verwenden, das gemizid, bakterizid, bakteriostatisch, fungizid, viruzid, virustatisch und/ oder allgemein mikrobiozid wirkt. Insbesondere sind gemäß der vorliegenden Erfindung solche Stoffe geeignet die ausgewählt werden aus der Gruppe Resorcinol, Iod, Iod-Povidon, Chlorhexidin, Benzalkoniumchlorid, Benzoesäure, Benzoylperoxid oder Cethylpyridiniumchlorid. Darüber hinaus sind als Antiseptika insbesondere auch antimikrobiellen Metalle zu verwenden. Als antimikrobielle Metalle können insbesondere Silber, Kupfer oder Zink sowie deren Salze, Oxide oder Komplexe in Kombination oder alleine verwendet werden.

Als pflanzliche wundheilungsfördernde Substanzen oder Substanzgemische oder Pflanzenextrakte sind im Zusammenhang mit der vorliegenden Erfindung insbesondere Extrakte der Kamille, Hamamelis-Extrakte z.B. Hamamelis virgina, Calendula-Extrakt, Aloe-Extrakt z.B. Aloe vera, Aloe barbadensis, Aloe ferox oder Aloe vulgaris, Grüntee-Extrakte, Meeresalgen-Extrakt z.B. Rotalgen- oder Grünalgen-Extrakt, Avocado-Extrakt, Myrre-Extrakt z.B. Commophora molmol, Bambus-Extrakte sowie Kombinationen hiervon zu verwenden. Hierbei sind erfindungsgemäß besonders Extrakte der Blätter, Blüten, Stengel oder Wurzeln der Pflanzen oder Kombinationen hiervon zu verstehen.

In einer weiteren Ausführung kann auch vorgesehen sein, das dass Flächengebilde oder die Wundkontaktschicht mindestens eine zweite von der ersten verschiedene wundheilungsfördernde Substanz umfasst.

Die wundheilungsfördernden Substanzen, die einzeln oder als Gemisch verschiedener wundheilungsfördernder Substanzen eingesetzt werden können, sind insbesondere bis zu 20 Gew.-%, bevorzugt zu 10 Gew.%, insbesonder bis zu 5 Gew.-%, insbesondere bis zu 3 Gew.-% und ganz besonders bevorzugt mehr als 0,01 Gew.-% bezogen auf das Gesamtgewicht des Flächengebildes oder der Wundkontaktschicht enthalten, wobei sich die Gewichtsangaben auf die Reinsubstanz bezieht.

Wie oben bereits angeführt handelt es sich bei einem medizinischen Flächengebilde um eine Wundkontaktschicht. Hierunter ist eine Schicht zu verstehen, die im anwendungsgerechten Zustand der Schicht in direktem Kontakt mit einer Wunde und oder einer Wunde umgebenden Haut liegt. Hierbei weist eine Wundkontaktschicht alle Merkmale des oben aufgeführten Flächengebildes, insbesondere des Fasermaterials, der Partikel und/ oder der wundheilungsfördernden Substanz auf. Damit ist eine Wundkontaktschicht umfassend mindestens eine erste Schicht aus einem Fasermaterial mit einer ersten, zu einer im Gebrauch dem Körper zugewandten Oberseite und einer zweiten im Gebrauch dem Körper abgewandten Oberseite, und mindestens eine wundheilungsfördernde Substanz, die in Form von Partikeln vorliegt, wobei die wundheilungsfördernde Substanz in Gegenwart von wässriger Flüssigkeit freigesetzt wird und die Partikel mit dem Fasermaterial verbunden sind, ebenfalls Gegenstand der vorliegenden Erfindung. Bei dieser Wundkontaktschicht handelt es sich insbesondere um eine nicht klebende und/ oder nicht wundverklebende Wundkontaktschicht.

In einer bevorzugten Ausführung der Wundkontaktschicht kann vorgesehen sein, dass die Wundkontaktschicht selbst eine Oberflächenbehandlung erfahren hat, damit die Wundkontaktschicht nicht wundverklebend ist. Es kann jedoch auch vorgesehen sein, dass die Wundkontaktschicht durch die Wahl des Fasermaterials oder die Wahl der Fasern nicht wundverklebend ist, das heißt, dass Fasern eingesetzt werden, die selbst oder im Verbund keine Wundverklebung zeigen. Hierbei hat sich es sich als besonders vorteilhaft erwiesen, die Wundkontaktschicht mehrschichtig auszugestalten.

Falls die Wundkontaktschicht mehrschichtig ist, hat es sich als besonders vorteilhaft erwiesen, wenn auf der zu einer zu behandelnden Oberfläche eines Patienten weisenden Oberseite der ersten Schicht des Fasermaterials eine zweite Schicht aus einem Fasermaterial angeordnet, die insbesondere nicht wundverklebend ist. Insbesondere umfasst diese zweite Schicht Fasern, die selbst oder im Verbund nicht wundverklebend sind.

Gemäß einem weiterführenden Gedanken umfasst die Erfindung auch eine mehrschichtige Wundauflage, die mindestens ein oben beschriebenes medizinisches Flächengebilde oder eine oben beschriebene Wundkontaktschicht umfasst. Diese Wundauflage umfasst mindestens ein oben beschriebenes medizinisches Flächengebilde oder eine oben beschriebene Wundkontaktschicht und eine weitere Schicht, insbesondere eine Trägerschicht auf der zweiten im Gebrauch dem Körper abgewandten Oberseite des Flächengebildes oder der Wundkontaktschicht insbesondere zur Befestigung auf der Haut eines Patienten, und eine absorbierende Schicht zur Aufnahme von Wundflüssigkeiten. Hierbei weist eine erfindungsgemäße Wundauflage alle Merkmale des oben aufgeführten Flächengebildes oder der Wundkontaktschicht, insbesondere des Fasermaterials, der Partikel und/ oder der wundheilungsfördernden Substanz auf. Bei dieser Wundauflage handelt es sich insbesondere um eine nicht klebende und/ oder nicht wundverklebende Wundauflage.

In einer Ausführung der vorliegenden Erfindung, umfasst eine mehrschichtige Wundauflage eine Wundkontaktschicht, die mindestens eine erste Schicht aus einem Fasermaterial mit einer ersten im Gebrauch dem Körper zugewandten Oberseite und einer zweiten im Gebrauch dem Körper abgewandten Oberseite, und mindestens eine wundheilungsfördernde Substanz, die in Form von Partikeln vorliegt, wobei die wundheilungsfördernde Substanz in Gegenwart von wässriger Flüssigkeit, insbesondere Wundexsudat, freigesetzt wird und die Partikel mit dem Fasermaterial verbunden sind, und eine auf der zweiten Oberseite angeordnete absorbierende Schicht wobei weiterhin bevorzugt auf der wundabgewandten Oberseite der absorbierenden Schicht eine Trägerschicht angeordnet ist. Eine solche Wundauflage weist auch besonders vorteilhaft eine Verteilerschicht zwischen der Trägerschicht und der absorbierenden Schicht oder zwischen der absorbierenden Schicht und der Trägerschicht auf. Insbesondere umfasst die Wundauflage eine absorbierende Schicht aus einem hydrophilen Polyurethan-Schaum. Durch die Verteilerschicht wird erreicht, dass eine Verteilung der aufgenommen Wundflüssigkeiten über die gesamte Fläche der Wundauflage insbesondere oberhalb der absorbierenden Schicht ermöglicht wird, das heißt das die Aufnahme der Wundflüssigkeiten nicht nur in z-Richtung (von der Wunde weg in Richtung Trägerschicht), sondern auch in x-y-Richtung (über die Fläche der Wundauflage) erfolgt.

Als Trägerschicht kann jedes heute in der modernen Wundbehandlung übliches, als Trägerschicht einzusetzendes Material Verwendung finden. Als im anwendungsgerechten Zustand der Wundauflage als äußere Lage angeordnete Trägerschicht kann insbesondere eine flüssigkeitsdichte Folie, ein Nonwoven oder eine elastisches Schaummaterial verwendet werden. Besonders bevorzugt sind flüssigkeitsdichte Folien, die zudem wasserdampfdurchlässig sind. Diese Folien können aus einer Vielzahl an Polymermaterialien gefertigt sein. Als besonders geeignet haben sich hier flüssigkeitsdichte und wasserdampfdurchlässige Folien aus Polyester, Copolyester, Polyurethan, Polyetherpolyurethan, Polyesterpolyurethan, Polypropylen, Polyethylen oder Polyamid gezeigt.

Besonders vorteilhaft haben sich unilaminare Folien mit einer Dicke von 20 bis 150 µm herausgestellt. Darüber hinaus haben sich Filme, die eine Wasserdampfdurchlässigkeit von mindestens 1000 g/ m²/ 24 Std. und vorzugsweise von mindestens 1200 g/ m²/ 24 Std. (gemessen nach DIN EN 13726) als besonders hautfreundlich herausgestellt. In besonders bevorzugten Ausführungsformen weisen diese Filme einen feuchtigkeitsdichten, klebenden Randabschnitt auf. Dieser Randabschnitt gewährleistet, dass die Wundauflage auf der Haut appliziert und fixiert werden kann. Darüber hinaus ist sichergestellt, dass keine Flüssigkeit zwischen der Folie und der die zu behandelnden Fläche umgebenden Haut austreten kann. Hierbei können alle bekannten feuchtigkeitsresistenten Klebstoffe Anwendung finden. Als besonders bevorzugt sind solche Klebstoffe zu betrachten, die in einem dünnen Auftrag von 20 bis 35 g/ m² zusammen mit dem Film eine Wasserdampfdurchlässigkeit von mindestens 800 g/ m²/ 24 Std. und vorzugsweise von mindestens 1000 g/ m²/ 24 Std. (gemessen nach DIN EN 13726) aufweisen.

Zur Bildung einer absorbierenden Schicht können eine Vielzahl an Materialien eingesetzt werden. Hierbei sind insbesondere solche Materialien zu nennen, die in der feuchten Wundtherapie angewendet werden können. Ganz besonders sind jedoch solche absorbierende Schichten bevorzugt, die sowohl Wundsekret aufnehmen und somit absorbierend wirken als auch Feuchtigkeit an die Wunde abgeben. Gemäß der vorliegenden Erfindung umfasst die Wundauflage als absorbierende Schicht einen hydrophilen Polymer-Schaum.

Wird als absorbierende Schicht ein hydrophiler Polymerschaum verwendet, so hat sich gezeigt, dass sich die Wundauflage besonders gut an eine zu behandelnde Wunde anpasst. Als Polymerschäume sind hierbei insbesondere hydrophile Polyurethan-Schäume geeignet. Dem gemäß umfasst eine besonders bevorzugte Wundauflage eine absorbierende Schicht aus einem hydrophilen Polyurethan-Schaum. Diese Polyurethan-Schäume weisen eine freie Absorption von mindestens 10 g/ g, insbesondere mindestens 12 g/ g und ganz besonders bevorzugt von mindestens 15 g/ g auf, wobei die freie Absorption gemäß der DIN-EN 13726-1 (2002) bestimmt wird. Weiterhin bevorzugt weisen diese Schäume eine Porengröße von durchschnittlich weniger als 1000 µm, insbesondere 200 bis 1000 µm und ganz besonders bevorzugt 200 bis 700 µm auf. Hierbei kann vorgesehen sein, dass die Porengröße an einer ersten Oberfläche der absorbierenden Schicht im Wert verschieden ist von der Porengröße einer zweiten Oberfläche der absorbierenden Schicht. Weiterhin bevorzugte hydrophile Polyurethan-Schäume weisen eine Dichte von weniger als 150 kg/ m³, insbesondere weniger als 140 kg/ m³ und ganz besonders bevorzugt 70 bis 120 kg/ m³ auf.

Im Zusammenhang mit einem weiteren Gedanken ist damit auch die Verwendung eines Flächengebildes, das mindestens eine erste Schicht aus einem Fasermaterial mit einer ersten im Gebrauch dem Körper zugewandten Oberseite und einer zweiten im Gebrauch dem Körper abgewandten Oberseite,, und mindestens eine wundheilungsfördernde Substanz umfasst, die in Form von Partikeln vorliegt, wobei die wundheilungsfördernde Substanz in Gegenwart von wässriger Flüssigkeit freigesetzt wird und die Partikel mit dem Fasermaterial verbunden sind, zur Herstellung eines Mittels zur Behandlung von Wunden, insbesondere zur Herstellung einer Wundauflage zur Behandlung von Wunden, insbesondere Chronische Wunden, Gegenstand der vorliegenden Erfindung.

In einer besonderen Ausgestaltung der Erfindung ist weiterhin vorgesehen, dass eine Wundkontaktschicht oder eine Wundauflage in einer Verpackung angeordnet ist. Insbesondere ist dabei vorgesehen, dass die Verpackung eine sterile Verpackung ist. In einer weiteren besonderen Ausgestaltung der Erfindung ist vorgesehen, dass ein Set umfassend eine Wundkontaktschicht der beschriebenen Art und eine separate Wundauflage in einer Verpackung angeordnet sind. Insbesondere ist dabei auch vorgesehen, dass die Verpackung eine sterile Verpackung ist. In einer besonders bevorzugten Ausführung dieses Systems liegt in der Verpackung oder in der sterilen Verpackung jeder einzelne Bestandteil oder jede Gruppe von Bestandteilen jeweils separat in Unterverpackungen vor. Es kann auch vorgesehen sein, dass jede Unterverpackung eine sterile Unterverpackung ist.

Hervorzuheben ist an dieser Stelle, dass die hier aufgeführten Merkmale der alternativen oder bevorzugten Ausgestaltungen der Erfindungen nicht auf die einzelnen Alternativen zu beschränken sind. Im Zusammenhang mit der vorliegenden Erfindung ist es vielmehr der Fall, dass eine Kombination von Ausgestaltungen bzw. eine Kombination jedes Einzelmerkmals einer alternativen Form mit Merkmalen einer anderen alternativen Ausgestaltung ebenso als erfindungsgemäßer Gegenstand zu sehen ist. Ebenso wenig soll die Erfindung durch die nachfolgende Beschreibung der Zeichnungen reduziert verstanden sein.

Nachstehend wird die Erfindung unter Bezugnahme auf Zeichnungen erläutert. In den Zeichnungen zeigt:
Figur 1: Wundkontaktschicht im Querschnitt
Figuren 2a und 2b: Faser mit wundheilungsfördernden Partikeln
Figur 3: Wundauflage im Querschnitt

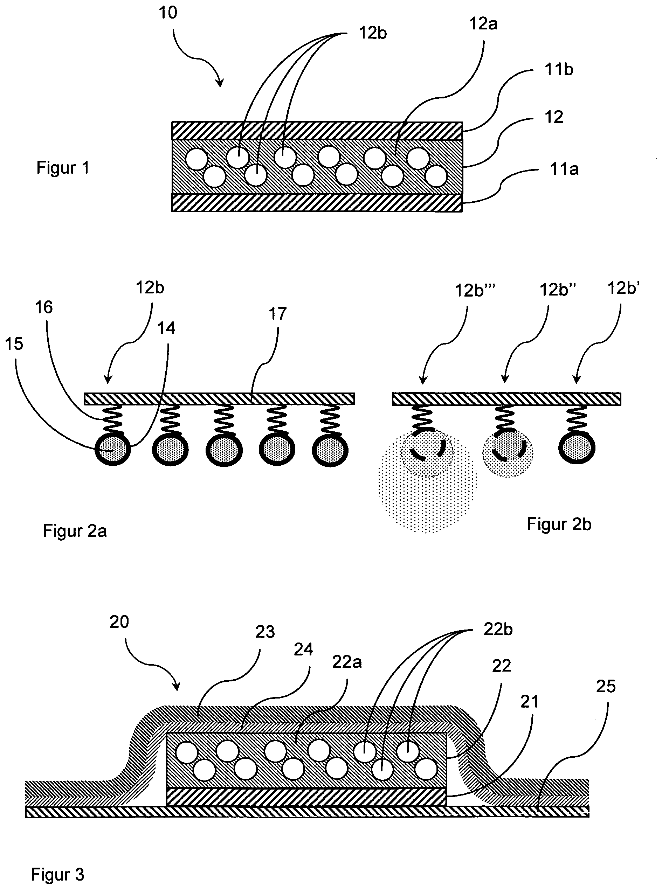

Mit Figur 1 ist ein Flächengebilde (10) als nicht wundverklebende Wundkontaktschicht gezeigt, das aus einer ersten Schicht (12) besteht, die zwischen einer angrenzenden zweiten Schichten (11a) und einer gegenüber liegenden dritten Schicht (11b) angeordnet ist. Die beiden Schichten (11a, 11b) bestehen aus einem hydrophoben Polyestervlies, das keine Wundverklebung zeigt. Die zentrale Schicht (12) ist symmetrisch zwischen den beiden äußeren Schichten (11a, 11b) angeordnet. Die zentrale Schicht (12) besteht aus einem Stapelfaservlies (12a) in das gleichmäßig feste Partikel (12b) einer wundheilungsfördernder Substanz verteilt sind. Das thermisch oberflächenverdichtete Vlies (12a) besteht aus ersten hydrophilen Fasern und zweiten Fasern, die als Bindefasern dienen. Die Partikel (12b) sind wie mit Figur 2a schematisch dargestellt auf den einzelnen Fasern (17) angeordnet und mit den Fasern verbunden (die Verbindung (16) ist schematisch dargestellt). Auf den einzelnen Fasern des Vlieses sind demnach mittels eines thermischen Prozesses aufgebrachte Partikel enthalten. Die Partikel (12b) sind als Kern-Hülle-Partikel ausgeführt und bestehen aus einem Kern (15) und einer Hülle (14), über die die Partikel mit den Fasern verbunden sind. Weiterhin ist mit Figur 2b die zeitliche Verzögerung der wundheilungsfördernden Substanz aus einer Partikel (12b) dargestellt. Zu einem ersten Zeitpunkt (12b'), in dem kein Wundexsudat in der näheren Umgebung der Partikel vorliegt, liegt die Partikel (12b) wie beschrieben an der Faser gebunden vor. Zu einem zweiten Zeitpunkt (12b"), dies ist in der bestimmungsgemäßen Verwendung der Wundkontaktschicht auf einer nässenden Wunde kurz nach der Applikation der Wundkontaktschicht, wird die Hülle der Partikel (12b) für die wundheilungsfördernde Substanz durchlässig, so dass die Substanz an die Wunde abgegeben wird. Zu einem späteren Zeitpunkt (12b"') wird in Gegenwart von vermehrtem Wundsekret weitere wundheilungsfördernde Substanz aus den Partikeln freigesetzt.

Mit Figur 3 ist eine nicht erfindungsgemäße nicht wundverklebende Wundauflage (20) als sogenannter Inselverband wiedergegeben. Die Wundauflage besteht aus einer ersten Schicht (22), die aus einem Stapelfaservlies (22a) aus wundheilungsfördernde Partikel (22b) umfassende Stapelfasern hergestellt ist. Das Vlies (22a) ist mittels eines Haftklebemittels (24) an einer Trägerschicht (23) festgelegt. Die im anwendungsgerechten Zustand der Wundauflage zu einer Wunde gewandte Seite des Vlieses ist mit einem hydrophoben Polyestervlies abgedeckt, so dass diese Wundauflage auch als nicht wundverklebende Wundauflage bezeichnet werden kann. Insgesamt ist die Wundauflage zum Schutz mit einem Releasepapier (25) abgedeckt.

## Patentansprüche

1. Mehrschichtige Wundauflage umfassend ein medizinisches Flächengebilde, worin das medizinische Flächengebilde eine Wundkontaktschicht ist und die Wundkontaktschicht mindestens eine erste Schicht aus einem Fasermaterial mit einer ersten im Gebrauch dem Körper zugewandten Oberseite und einer zweiten im Gebrauch dem Körper abgewandten Oberseite und mindestens eine wundheilungsfördernde Substanz, die in Form von Partikeln vorliegt, umfasst, wobei die Partikel mit dem Fasermaterial verbunden sind und die wundheilungsfördernde Substanz in Gegenwart von wässriger Flüssigkeit freigesetzt wird,
**dadurch gekennzeichnet, dass**
auf der zweiten Oberseite eine absorbierende Schicht angeordnet ist und die Wundauflage als diese absorbierende Schicht einen hydrophilen Polymer-Schaum umfasst.

2. Mehrschichtige Wundauflage nach Anspruch 1 **dadurch gekennzeichnet, dass** die Wundkontaktschicht eine nicht klebende und/oder nicht wundverklebende Wundkontaktschicht ist.

3. Mehrschichtige Wundauflage nach Anspruch 2 **dadurch gekennzeichnet, dass** die Wundkontaktschicht eine mehrschichtige Wundkontaktschicht ist und dass auf der zu einer zu behandelnden Oberfläche eines Patienten weisenden Oberfläche der ersten Schicht des Fasermaterials eine zweite Schicht aus einem Fasermaterial angeordnet ist, die nicht wundverklebend ist.

4. Mehrschichtige Wundauflage nach mindestens einem der vorangehenden Ansprüche **dadurch gekennzeichnet, dass** der hydrophile Polymerschaum ein hydrophiler Polyurethanschaum ist.

5. Mehrschichtige Wundauflage nach mindestens einem der vorangehenden Ansprüche **dadurch gekennzeichnet, dass** die Partikel aus einem Trägermaterial und der wundheilungsfördernden Substanz bestehen.

6. Mehrschichtige Wundauflage nach mindestens einem der vorgenannten Ansprüche **dadurch gekennzeichnet, dass** die Partikel einen Kern und eine Hülle aufweisen und die Partikel mittels der Hülle mit dem Fasermaterial verbunden sind.

7. Mehrschichtige Wundauflage nach mindestens einem der vorgenannten Ansprüche **dadurch gekennzeichnet, dass** das Trägermaterial oder die Hülle aus einem gelbildenden Polymer besteht.

8. Mehrschichtige Wundauflage nach mindestens einem der vorgenannten Ansprüche **dadurch gekennzeichnet, dass** das gelbildende Polymer ausgewählt wird aus der Gruppe der Polyacrylate, Alginate, Chitin oder dessen Derivate, Cellulose oder deren Derivate.

9. Mehrschichtige Wundauflage nach mindestens einem der vorgenannten Ansprüche **dadurch gekennzeichnet, dass** die wundheilungsfördernde Substanz ausgewählt ist aus der Gruppe der Vitamine oder Provitamine, Carotinoide, Analgetika, Antiseptika, Hämostyptika, Antihistaminika, antimikrobiellen Metalle oder deren Salze, pflanzlichen wundheilungsfördernden Substanzen oder Substanzgemische, Pflanzenextrakte, Enzyme, Wachstumsfaktoren, Enzyminhibitoren sowie Kombinationen hiervon.

10. Mehrschichtige Wundauflage nach mindestens einem der vorgenannten Ansprüche **dadurch gekennzeichnet, dass** die erste Schicht ein Nonwoven, ein Gestrick, ein Gewirk oder ein Gewebe ist.

11. Mehrschichtige Wundauflage nach mindestens einem der vorgenannten Ansprüche **dadurch gekennzeichnet, dass** das Fasermaterial als erste Fasern Stapelfasern aus synthetischen und/ oder natürlichen Polymeren enthält.

12. Mehrschichtige Wundauflage nach mindestens einem der vorgenannten Ansprüche **dadurch gekennzeichnet, dass** das Fasermaterial aus Viskosefasern, Cellulosefasern, Alginatfasern, Acetatfasern, Polyersterfasern, Polyamidfasern, Polyethylenfasern, Polypropylenfasern oder Kombinationen hiervon besteht.

13. Mehrschichtige Wundauflage nach mindestens einem der vorgenannten Ansprüche **dadurch gekennzeichnet, dass** das Fasermaterial aus Viskosefasern und/ oder Cellulosefasern besteht.

14. Mehrschichtige Wundauflage nach mindestens einem der vorangehenden Ansprüche **dadurch gekennzeichnet, dass** das Flächengebilde wasserstrahlvernadelt und/oder thermoverfestigt ist.

15. Mehrschichtige Wundauflage nach mindestens einem der vorgenannten Ansprüche **dadurch gekennzeichnet, dass** das Flächengebilde mindestens eine zweite von der ersten verschiedene wundheilungsfördernde Substanz umfasst.

16. Mehrschichtige Wundauflage nach mindestens einem der vorgenannten Ansprüche **dadurch gekennzeichnet, dass** die erste und die zweite wundheilungsfördernde Substanz in voneinander verschiedenen Partikeln vorliegen.

## Claims

1. Multilayer wound dressing comprising a medical fabric, the medical fabric being a wound contact layer and the wound contact layer comprising at least one first layer composed of a fibrous material having a first top side facing the body when in use and a second top side facing away from the body when in use and at least one wound-healing-promoting substance present in the form of particles, the particles being connected to the fibrous material and the wound-healing-promoting substance being released in the presence of aqueous liquid,
**characterized in that**
an absorbing layer is arranged on the second top side and that the wound dressing comprises a hydrophilic polymer foam as this absorbing layer.

2. Multilayer wound dressing according to Claim 1, **characterized in that** the wound contact layer is a non-adherent and/or non-wound-sticking wound contact layer.

3. Multilayer wound dressing according to Claim 2, **characterized in that** the wound contact layer is a multilayer wound contact layer and **in that** the surface of the first layer of the fibrous material, which surface is pointing to a surface of a patient that is to be treated, has arranged thereon a second layer composed of a fibrous material which is non-woundsticking.

4. Multilayer wound dressing according to at least one of the preceding claims, **characterized in that** the hydrophilic polymer foam is a hydrophilic polyurethane foam.

5. Multilayer wound dressing according to at least one of the preceding claims, **characterized in that** the particles consist of a carrier material and the woundhealing-promoting substance.

6. Multilayer wound dressing according to at least one of the preceding claims, **characterized in that** the particles have a core and a shell and the particles are connected to the fibrous material by means of the shell.

7. Multilayer wound dressing according to at least one of the preceding claims, **characterized in that** the carrier material or the shell consists of a gel-forming polymer.

8. Multilayer wound dressing according to at least one of the preceding claims, **characterized in that** the gel-forming polymer is selected from the group of polyacrylates, alginates, chitin or derivatives thereof, cellulose or derivatives thereof.

9. Multilayer wound dressing according to at least one of the preceding claims, **characterized in that** the wound-healing-promoting substance is selected from the group of vitamins or provitamins, carotenoids, analgesics, antiseptics, haemostyptics, antihistamines, antimicrobial metals or salts thereof, plant-based wound-healing-promoting substances or substance mixtures, plant extracts, enzymes, growth factors, enzyme inhibitors and also combinations thereof.

10. Multilayer wound dressing according to at least one of the preceding claims, **characterized in that** the first layer is a nonwoven, a weft-knitted fabric, a warp-knitted fabric or a woven fabric.

11. Multilayer wound dressing according to at least one of the preceding claims, **characterized in that** the fibrous material contains, as first fibres, staple fibres composed of synthetic and/or natural polymers.

12. Multilayer wound dressing according to at least one of the preceding claims, **characterized in that** the fibrous material consists of viscose fibres, cellulose fibres, alginate fibres, acetate fibres, polyester fibres, polyamide fibres, polyethylene fibres, polypropylene fibres or combinations thereof.

13. Multilayer wound dressing according to at least one of the preceding claims, **characterized in that** the fibrous material consists of viscose fibres and/or cellulose fibres.

14. Multilayer wound dressing according to at least one of the preceding claims, **characterized in that** the fabric is hydroentangled and/or thermoconsolidated.

15. Multilayer wound dressing according to at least one of the preceding claims, **characterized in that** the fabric comprises at least one second wound-healing promoting substance different from the first one.

16. Multilayer wound dressing according to at least one of the preceding claims, **characterized in that** the first and the second wound-healing-promoting substance are present in particles different from one another.

## Revendications

1. Pansement multicouche comprenant une structure plate médicale, dans lequel la structure plate médicale est une couche de contact avec la plaie et la couche de contact avec la plaie comprend au moins une première couche en un matériau fibreux comprenant un premier côté supérieur orienté vers le corps à l'usage et un deuxième côté supérieur opposé au corps à l'usage et au moins une substance favorisant la cicatrisation, qui se présente sous la forme de particules, les particules étant reliées avec le matériau fibreux et la substance favorisant la cicatrisation étant libérée en présence d'un liquide aqueux, **caractérisé en ce qu'**une couche absorbante est agencée sur le deuxième côté supérieur et **en ce que** le pansement comprend une mousse polymère hydrophile comme cette couche absorbante.

2. Pansement multicouche selon la revendication 1, **caractérisé en ce que** la couche de contact avec la plaie est une couche de contact avec la plaie qui ne colle pas et/ou qui n'adhère pas à la plaie.

3. Pansement multicouche selon la revendication 2, **caractérisé en ce que** la couche de contact avec la plaie est une couche de contact avec la plaie multicouche et **en ce qu'**une deuxième couche en un matériau fibreux, qui n'adhère pas à la plaie, est agencée sur la surface de la première couche du matériau fibreux orientée vers une surface à traiter d'un patient.

4. Pansement multicouche selon au moins l'une quelconque des revendications précédentes, **caractérisé en ce que** la mousse polymère hydrophile est une mousse de polyuréthane hydrophile.

5. Pansement multicouche selon au moins l'une quelconque des revendications précédentes, **caractérisé en ce que** les particules sont constituées par un matériau support et la substance favorisant la cicatrisation.

6. Pansement multicouche selon au moins l'une quelconque des revendications précédentes, **caractérisé en ce que** les particules comprennent un noyau et une enveloppe, et les particules sont reliées par l'enveloppe avec le matériau fibreux.

7. Pansement multicouche selon au moins l'une quelconque des revendications précédentes, **caractérisé en ce que** le matériau support ou l'enveloppe est constitué par un polymère formant un gel.

8. Pansement multicouche selon au moins l'une quelconque des revendications précédentes, **caractérisé en ce que** le polymère formant un gel est choisi dans le groupe constitué par les polyacrylates, les alginates, la chitine ou ses dérivés, la cellulose ou ses dérivés.

9. Pansement multicouche selon au moins l'une quelconque des revendications précédentes, **caractérisé en ce que** la substance favorisant la cicatrisation est choisie dans le groupe constitué par les vitamines ou les provitamines, les caroténoïdes, les analgésiques, les antiseptiques, les hémostyptiques, les antihistaminiques, les métaux antimicrobiens ou leurs sels, les substances ou mélanges de substances végétales favorisant la cicatrisation, les extraits végétaux, les enzymes, les facteurs de croissance, les inhibiteurs enzymatiques, ainsi que leurs combinaisons.

10. Pansement multicouche selon au moins l'une quelconque des revendications précédentes, **caractérisé en ce que** la première couche est un non-tissé, un tricot ou un tissu.

11. Pansement multicouche selon au moins l'une quelconque des revendications précédentes, **caractérisé en ce que** le matériau fibreux contient en tant que premières fibres des fibres discontinues en polymères synthétiques et/ou naturels.

12. Pansement multicouche selon au moins l'une quelconque des revendications précédentes, **caractérisé en ce que** le matériau fibreux est constitué par des fibres de viscose, des fibres de cellulose, des fibres d'alginate, des fibres d'acétate, des fibres de polyester, des fibres de polyamide, des fibres de polyéthylène, des fibres de polypropylène ou leurs combinaisons.

13. Pansement multicouche selon au moins l'une quelconque des revendications précédentes, **caractérisé en ce que** le matériau fibreux est constitué par des fibres de viscose et/ou des fibres de cellulose.

14. Pansement multicouche selon au moins l'une quelconque des revendications précédentes, **caractérisé en ce que** la structure plate est aiguilletée par jet d'eau et/ou thermo-solidifiée.

15. Pansement multicouche selon au moins l'une quelconque des revendications précédentes, **caractérisé en ce que** la structure plate comprend au moins une deuxième substance favorisant la cicatrisation différente de la première.

16. Pansement multicouche selon au moins l'une quelconque des revendications précédentes, **caractérisé en ce que** la première et la deuxième substance favorisant la cicatrisation se trouvent dans des particules différentes les unes des autres.
